(19) Europäisches Patentamt
European Patent Office
Office européen des brevets

(11) Publication number: 0 172 744
A2

# (12) EUROPEAN PATENT APPLICATION

(21) Application number: 85305868.3

(22) Date of filing: 16.08.85

(51) Int. Cl.[4]: C 07 D 241/46
A 61 K 31/495

(30) Priority: 16.08.84 NZ 209247

(43) Date of publication of application:
26.02.86 Bulletin 86/9

(84) Designated Contracting States:
AT BE CH DE FR GB IT LI LU NL SE

(71) Applicant: DEVELOPMENT FINANCE CORPORATION OF NEW ZEALAND
Development Finance Centre Corner Grey and Featherston Streets
Wellington(NZ)

(72) Inventor: Atwell, Graham John
37 Hawkins Street
Meadowbank Auckland 5(NZ)

(72) Inventor: Baguley, Bruce Charles
36a Olsen Avenue
Hillsborough Auckland 4(NZ)

(72) Inventor: Denny, William Alexander
165 Gossamer Drive
Pakuranga Auckland(NZ)

(72) Inventor: Rewcastle, Gordon William
24 Rothery Road
Manurewa Auckland(NZ)

(74) Representative: Senior, Janet et al,
Abel & Imray Northumberland House 303-306 High Holborn
London WC1V 7LH(GB)

## (54) Phenazinecarboxamide compounds.

(57) The novel class of phenazine derivatives of the present invention represented by the general formula (I):

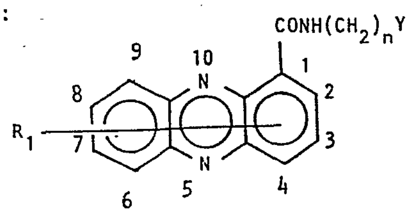

(I)

where $R_1$ represents H or up to three substituents, at positions selected from 2 to 4 and 6 to 9, wherein any two or all of the substituents may be the same or different and the substituents are selected from

lower alkyl radicals;
lower alkyl radicals substituted by one or more of the same or different substituents selected from hydroxy, amino and ether functions;
OH,
SH;
$OCH_2Ph$;
OPh;
$NO_2$;
halogen;
$CF_3$;
amino;
$NHSO_2R_2$, $NHCOR_2$, $NHCOOR_2$, $OR_2$ and $SR_2$ (where $R_2$ represents a lower alkyl radical which is unsubstituted or substituted by one or more of the same or different substituents selected from hydroxy, amino and ether functions); and
$CONH(CH_2)_{n'}Y'$ (where n' and Y' are as defined below), there being a maximum of one $CONH(CH_2)_{n'}Y'$ group; or any two of $R_1$ at adjacent positions represent -CH=CH-CH=CH- as part of an extra benzene ring or $-O-CH_2-O-$ (methylenedioxy) and the third of $R_1$ has any one of the meanings given above with the exception of an OH at position 2;

Y and Y', which may be the same or different, each represents $C(NH)NH_2$, $NHC(NH)NH_2$ or $NR_3R_4$, where each of $R_3$ and $R_4$, which may be the same or different, represents H or a lower alkyl radical unsubstituted or substituted by one or more of the same or different substituents selected from hydroxy, amino and ether functions, or $R_3$ and $R_4$, together with the nitrogen atom to which they are attached, form a heterocyclic ring; and

n and n', which may be the same or different, each represents an integer from 2 to 6; and the acid addition salts, 5- and 10- mono-N-oxides and 5,10-di-N-oxides thereof, possess antibacterial and antitumour properties.

EP 0 172 744 A2

The present invention relates to novel phenazine derivatives having antibacterial and antitumour activity, to methods of preparing the novel compounds, and to their use as antibacterial and antitumour agents. The present invention also relates to novel compounds useful as intermediates in the preparation of the phenazine derivatives of the invention, and to methods for their preparation.

Phenazines have long been known as natural products (J. M. Ingram and A. C. Blackwood, Adv. Appl. Microbiol. 13, 267 (1970)), including phenazine-1-carboxamide (oxychlororaphine), and methoxylated derivatives of phenazine-1-carboxylic acid (e.g. griseolutin).

Phenazine-1-carboxylic acid has been evaluated as an antitumour agent (V. P. Chernetskii et al, Onkologyia (Kiev), 5, 110 (1974), and O. A. Sidorik et al., Fiziol. Act. Veshch., 6, 92 (1974); Chem. Abstr. 83, 37610p and 22365p). Substituted phenazine-1-carboxamides have also been evaluated for antitumour activity, and found active against sarcoma 180 (K. Katagiri et al, Shion. Kenkyu. Nempo, 17, 127 (1967); Chem. Abstr. 68, 20644v and see also Chem. Abstr., 66, 1283c; 66, 36499w; 68, 20645w; 68, 20815b; 72, 220460q.).

Phenazine-1-carboxamides bearing a tetrazole sidechain have been evaluated as antiallergy agents (U.S. Patent 4,377,579 for "N-(tetrazol-5-yl)phenazine-1-carboxamides" issued to Minnesota Mining and Manufacturing Company; Chem. Abstr. 99, 22499r (1983)).

3,4-Benzophenazine-1-carboxamides bearing a variety of sidechains (including $CH_2CH_2N(Me)_2$), have been evaluated for antimicrobial activity: K. Kobayashi et al, Yakugaku Zasshi, 103, 165 (1983); Chem. Abstr. 99, 22428s (1983).

Phenazine-1,9-dicarboxamides bearing $(CH_2)_3NR_1R_2$ sidechains, i.e. 1,9-phenazine-bis(dialkylaminocarboxamides), have been evaluated for

antimalarial activity. (S.N. Sawhney and D.W. Boykin, 68 524 (9179).

The novel class of phenazines derivatives of the present invention is represented by the general formula (I):

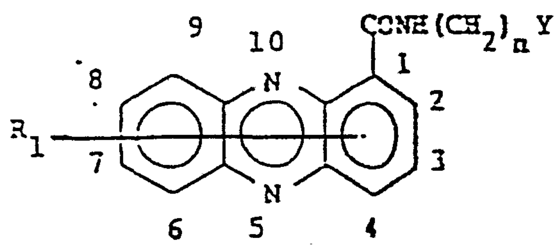

(I)

where $R_1$ represents H or up to three substituents, at positions selected from 2 to 4 and 6 to 9, wherein any two or all of the substituents may be the same or different and the substituents are selected from

lower alkyl radicals;

lower alkyl radicals substituted by one or more of the same or different substituents selected from hydroxy, amino and ether functions;

OH,

SH;

$OCH_2Ph$;

OPh;

$NO_2$;

halogen;

$CF_3$;

amino;

$NHSO_2R_2$, $NHCOR_2$, $NHCOOR_2$, $OR_2$ and $SR_2$ (where $R_2$ represents a lower alkyl radical which is unsubstituted

0172744

or substituted by one or more of the same or different substituents selected from hydroxy, amino and ether functions); and

$CONH(CH_2)_{n'}Y$ (where n' and Y' are as defined below), there being a maximum of one $CONH(CH_2)_{n'}Y'$ group;

or any two of $R_1$ at adjacent positions represent -CH=CH-CH=CH- as part of an extra benzene ring or $-O-CH_2-O-$ (methylenedioxy) and the third of $R_1$ has any one of the meanings given above with the exception of an OH at position 2;

Y and Y', which may be the same or different, each represents $C(NH)NH_2$, $NHC(NH)NH_2$ or $NR_3R_4$, where each of $R_3$ and $R_4$, which may be the same or different, represents H or a lower alkyl radical unsubstituted or substituted by one or more of the same or different substituents selected from hydroxy, amino and ether functions, or $R_3$ and $R_4$, together with the nitrogen atom to which they are attached, form a heterocyclic ring; and

n and n', which may be the same or different, each represents an integer from 2 to 6;

and the acid addition salts, 5- and 10- mono-N-oxides and 5,10-di-N-oxides thereof.

When $R_1$, $R_2$, $R_3$, $R_4$ or $R_6$ (referred to below) represent lower alkyl, or in the case of $R_6$ lower alkoxy, the group may contain from 1 to 5 carbon atoms.

As indicated above, Y and Y' may be the same or different and n and n' may be the same or different.

Thus, when present, the $CONH(CH_2)_{n'}Y'$ group may be the same as or different from the $CONH(CH_2)_nY$ group in position 1, but is usually the same.

An amino substituent in formula I may be unsubstituted or, for example, substituted by one or two lower alkyl groups (where lower alkyl has the meaning given above), especially by one or two methyl groups. Thus, for example, an amino substituent of a lower alkyl radical represented by $R_1$ and/or $R_2$ may be $NH_2$, $NHCH_3$ or $N(CH_3)_2$.

An ether function as substituent of a lower alkyl radical represented by $R_1$ and/or $R_2$ is preferably a lower alkoxy radical (where lower alkoxy has the meaning given above), more especially a methoxy group.

A heterocyclic radical represented by $R_3$ and $R_4$ and the nitrogen atom to which they are attached may, if desired, contain, for example, an additional hetero atom and, for example, may be 5- or 6-membered. An example is a morpholino group.

There should especially be mentioned compounds in which there is one $R_1$ substituent at position 9 and only one $CONH(CH_2)_nY$ group in the molecule (in position 1).

There should also especially be mentioned compounds in which two of $R_1$ at positions 8 and 9 represent -CH=CH-CH=CH-, completing an extra benzene ring.

Compounds in which Y represents $N(CH_3)_2$ should especially be mentioned.

A preferred class of compound of the above formula (I) is that where $R_1$ represents one of lower alkyl optionally substituted with hydroxy, amino or ether functions, OH, SH, $OCH_2Ph$, OPh, $NO_2$, halogen, $CF_3$, $NH_2$, or $NHSO_2R_2$, $NHCOR_2$, $NHCOOR_2$, $OR_2$ or $SR_2$ (where $R_2$ is lower alkyl optionally substituted with hydroxy, amino or ether functions) at position 9, or where two of $R_1$ at positions 8 and 9 represent -CH=CH-CH=CH- as part of an extra benzene ring; Y represents $N(CH_3)_2$; and n is from 2 to 6.

The compounds of formula (I) have antibacterial and antitumour activity, and are useful as antibacterial and antitumour agents.

The compounds of formula (I) form pharmaceutically acceptable addition salts with both organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic, and the like.

The compounds of general formula (I) and the acid addition salts, 5- and 10- mono-N-oxides and 5,10-di-N-oxides thereof may be prepared by a process which comprises coupling a substituted phenazine of the general formula (II),

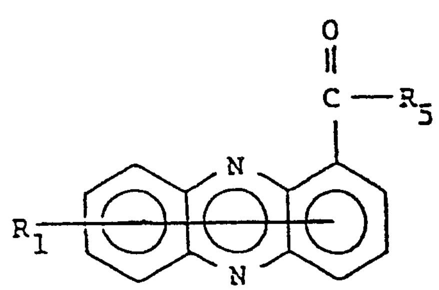

(II)

where $R_1$ is defined as above but additionally can represent $COR_5'$ at the positions 2-4 and 6-9, and $R_5$ and $R_5'$, which may be the same or different, each represents Cl, Br, $OCH_4$-p-$NO_2$, O-(1,N-benzotriazole), 1-N-imidazole, or O-(2-N-methylpyridinium) salts, or the 5- or 10- mono-N-oxide or 5,10-di-N-oxide thereof, with a primary alkyl amine of the general formula (III),

$$NH_2(CH_2)_nY \qquad (III)$$

where n and Y are as defined above, and, if desired, converting a compound of the invention into another such compound, for example a compound of formula (I) into an acid addition salt thereof or vice-versa.

As will be appreciated, when a $COR_5'$ group is present in the compound of formula II and the final compound of formula I should contain a group $CONH(CH_2)_{n'}Y'$ which is different from the $CONH(CH)_2)_nY$ group in position 1, two different amines of the formula III are employed.

The coupling reaction is desirably performed in an anhydrous solvent (e.g. chloroform, dimethylsulphoxide or N-methylpyrrolidone, but preferably dichloromethane or dimethylformamide) buffered with a tertiary amine, preferably triethylamine. The reaction is conveniently performed at temperatures in the range of from 0°C to 50°C, with the preferred temperature being 20°C.

The acid addition salts of the compounds of formula (I) may be prepared by contacting the free base form with an equivalent amount of the desired acid in the conventional manner. The free base forms may be regenerated by treating the salt form with a base. For example, dilute aqueous base solutions may be utilized. Dilute aqueous potassium hydroxide, potassium carbonate, ammonia, and sodium bicarbonate solutions are suitable for this purpose. The free base forms differ from their respective salt forms somewhat in certain physical properties such as solubility in polar solvents, but, in general, the salts are otherwise equivalent to their respective free base forms for the purposes of the invention.

The primary alkyl amines of the general formula (III) are known compounds and are commercially available or preparable by methods described in the literature. Examples of such compounds include N,N-dimethyl-1,2-ethanediamine (N,N-dimethylethylenediamine), N,N-diethyl-1,2-ethanediamine, N,N-dimethyl-1,3-propanediamine, N,N-dimethyl-1,4-butanediamine, N,N-dimethyl-1,5-pentanediamine, N-(2-hydroxyethyl)-1, 2-ethanediamine (2-(2-aminoethylamino)-ethanol), N-methyl-N-(2-hydroxyethyl)-1,2-ethanediamine, 2-aminoethylguanidine $NH_2(CH_2)_2NHC(NH)CH_2$, and 3-aminopropionamidine $NH_2(CH_2)_2C(NH)NH_2$. The two last-mentioned compounds may be prepared according to P. L. Barker, P. L. Gendler, and H. Rapoport, J. Org. Chem., 46, 2455 (1981).

The substituted phenazines of formula (II) are novel compounds useful as intermediates in the preparation of the compounds of formula (I), and accordingly the present invention also provides the compounds represented by the general formula (II),

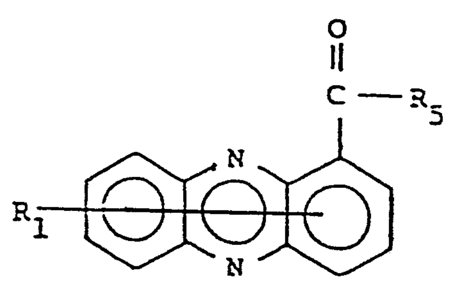

(II)

where $R_1$ is defined as for formula (I) but additionally can represent $COR_5'$ at positions 2-4 and 6-9, and $R_5$ and $R_5'$ each represents Cl, Br, $OC_6H_4$-p-$NO_2$, O-(1-N-benzotriazole), 1-N-imidazole, or O-(2-N-methylpyridinium) salts,

and the 5- and 10- mono-N-oxides and 5,10-di-N-oxides thereof.

Substituted phenazine-1-carboxylic acids, which are compounds of general formula (II) where $R_5$ is OH and $R_1$ is defined as in formula (I) but is not $NO_2$, and additionally can represent COOH, at positions 6-9, may be prepared by the process outlined in Scheme I. (S.R. Challand, R. B. Herbert and F. G. Holliman, J. Chem. Soc. Chem. Comm., 1970 (1423).

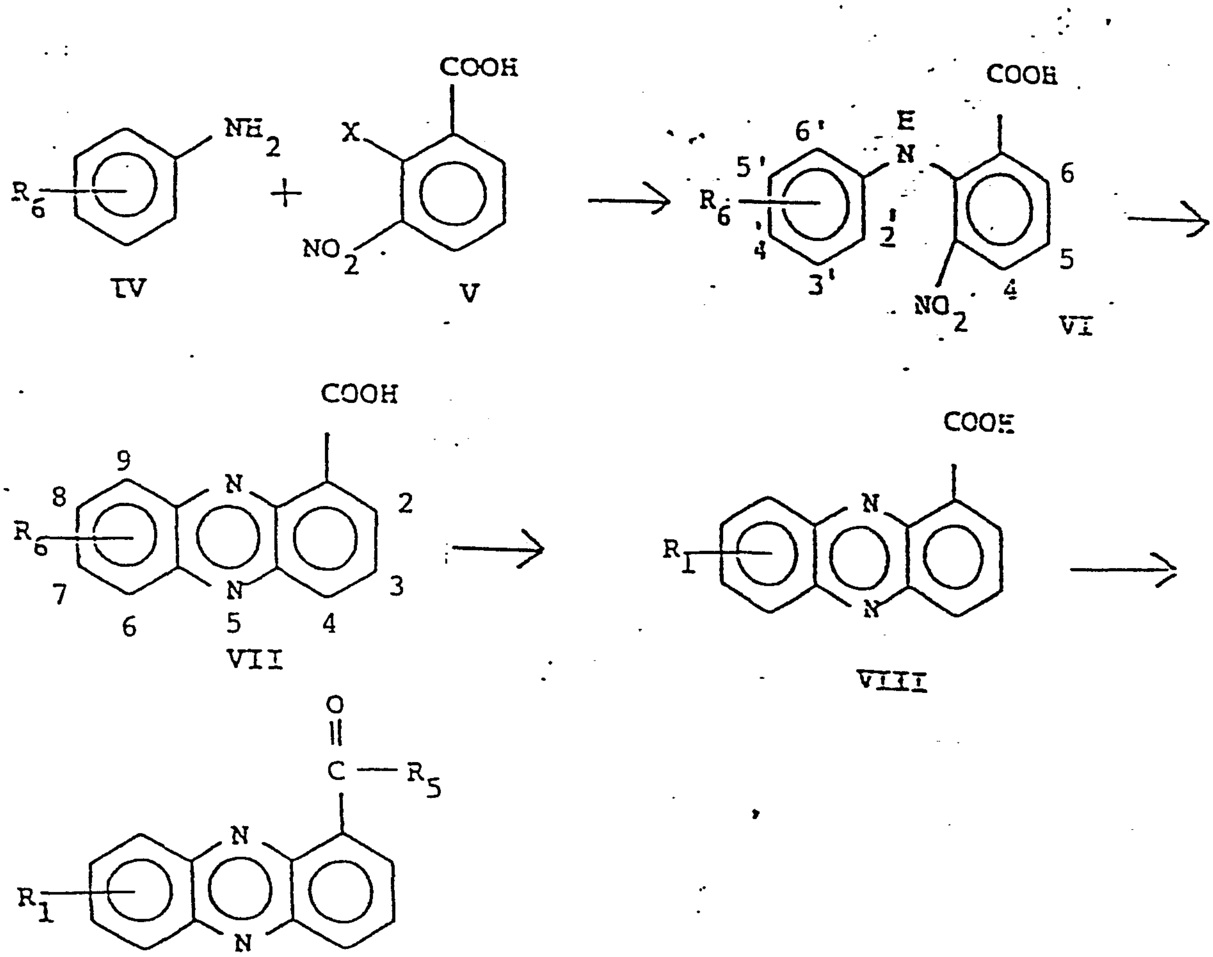

IV V VI

VII VIII

II

SCHEME I

0172744

In Scheme I, $R_6$ represents H or up to three of the groups halogen, SH, $OCH_2Ph$, OPh, $CF_3$, COOH, $NO_2$, lower alkyl or lower alkoxyl optionally substituted with ~~either~~ suitably-protected hydroxy and/or suitably-protected amino function(s), $NHSO_2R_2$, $NHCOR_2$, $NHCOOR_2$ or $SR_2$ (where $R_2$ is as defined above) or two of $R_6$ at adjacent positions represent -CH=CH-CH=CH- as part of an extra benzene ring or $-O-CH_2-O-$ (methylenedioxy); and X is Cl, Br or I.

Condensation of substituted anilines (IV) with 3-nitro-2-halobenzoic acids (V), preferably under Cu catalysis, provides the N-phenylanthranilic acids (VI), which undergo reductive cyclization, preferably with $NaBH_4$ in alkali, to give the phenazine 1-carboxylic acids (VII), where $R_6$ represents substituents in the 6,7,8 and/or 9 positions. Compounds (IV) where $R_6$ is $NO_2$ provide acids (VII) where $R_6$ is $NH_2$, due to concomitant reduction. Compounds of formula (VII) where $R_6$ represents $R_1$, where $R_1$ is as defined for formula (I), are also compounds of formula (VIII). Otherwise, or in addition, by appropriate substitution or replacement reactions compounds of formula (VII) wherein $R_6$ is as defined above can be converted to compounds of formula (VIII) where $R_1$ is as defined for formula (I) but additionally can represent COOH at positions 6-9. The compounds of formula (VIII) are activated into compounds of formula (II) as hereinafter described.

When $R_6$ in Scheme I is a potentially displaceable group (e.g. halogen, SH, $OCH_2Ph$, OPh, $NHSO_2R_2$, $NHCOR_2$, $NHCOOR_2$ or $SR_2$) especially in the 2'-position, ring closure can occur to varying extents by loss of $R_6$, to provide a mixture of the parent acid (VII, $R_6$ = H) together with the desired product. Because such mixtures are difficult to separate on a large scale, alternative processes for the synthesis of such compounds have been found.

One such alternative process is outlined in Scheme II,

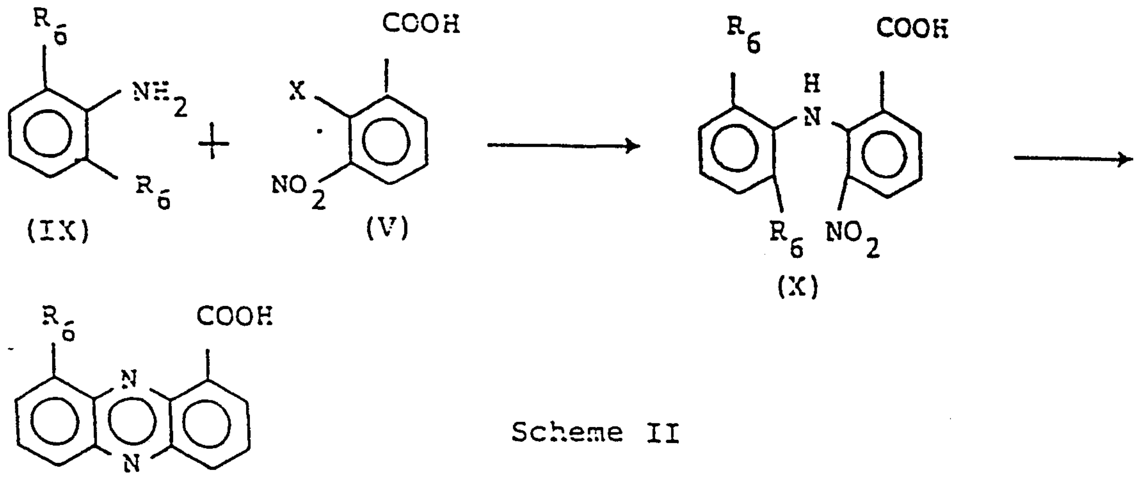

(IX) (V) (X)

(VIIA)

Scheme II

In Scheme II, $R_6$ and X are as defined above, subject to $R_6$ being restricted to the specified positions and the two $R_6$ groups may be the same or different. This process employs preferably symmetrical 2,6-disubstituted anilines (IX), with subsequent ring closure by loss of one group $R_6$ to provide 9-substituted phenazine-1-carboxylic acids (VIIA) unequivocally. While very useful, this process is, however, limited to potentially- displaceable groups $R_6$ of small size (e.g. $OCH_3$,F) in order for the reaction to form (X) to proceed in reasonable yield, and is applicable only to the unequivocal synthesis of 9-substituted phenazine acids. A more versatile process employing a displaceable group is outlined in Scheme III, and this process also forms part of the present invention.

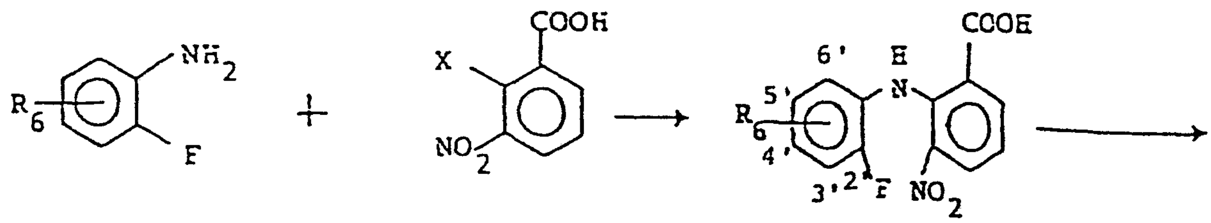

(XII) (V) (XIII)

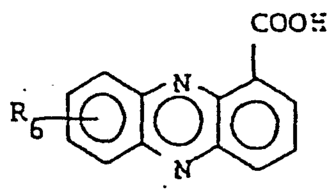

(VII)

Scheme III

In Scheme III, $R_6$ and X are as defined above. Reaction of fluorinated, substituted anilines (XII) with 3-nitro-2-halobenzoic acids (V) provides 2'-fluorinated derivatives (XIII). On reductive ring closure as outlined above, preferential loss of the fluorine leads to unequivocal ring closure, and this process is especially suitable for the unequivocal synthesis of 6- and 8-substituted phenazine-1-carboxylic acids.

The compounds represented by the general formulae (X) in Scheme II and (XIII) in Scheme III are novel compounds useful in the preparation of the compounds of formula (I), and they accordingly form part of the present invention.

The preparation of phenazine-1-carboxylic acids with at least one substituent in the 2-, 3- or 4-positions by the general process of Scheme I requires the preparation of highly-substituted benzoic acids (XVIII), which are best prepared <u>via</u> the isatins (XVI) as depicted in Scheme IV.

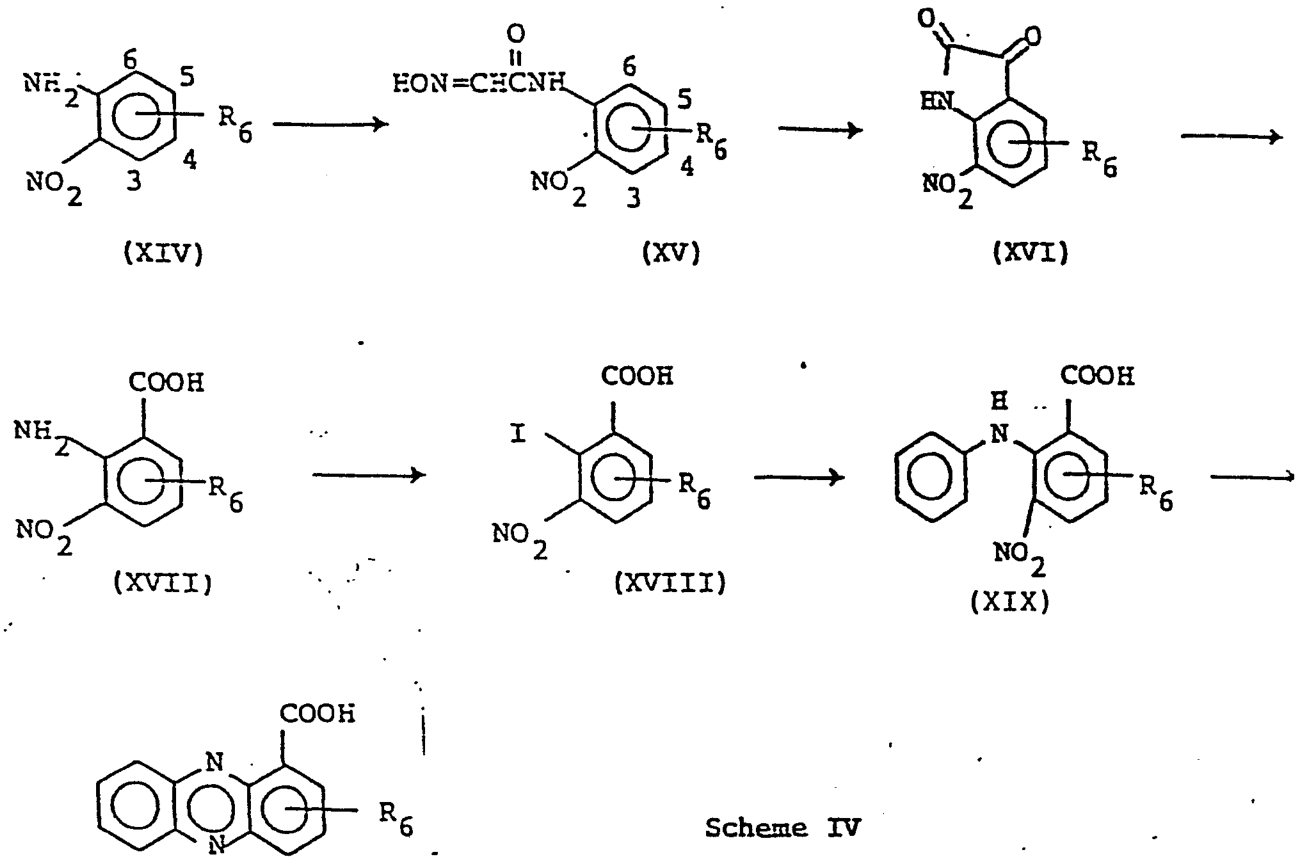

(XIV) (XV) (XVI)

(XVII) (XVIII) (XIX)

(VIIB)

Scheme IV

In Scheme IV, $R_6$ is as defined above, except that no substituents are permitted at the 6-positions in compounds XIV and XV. Reaction of suitably-substituted 2-nitroanilines (XIV) with chloral hydrate and hydroxylamine gives the isonitrosoacetanilides (XV), which can be cyclized under acid conditions to give the substituted isatins (XVI). Oxidative ring opening gives the anthranilic acid derivatives (XVII), which in turn give the iodoacids (XVIII) under Sandmeyer conditions. Following the procedures described in Scheme I, Scheme II or Scheme III then leads to substituted phenazine-1-carboxylic acids (VIIB) optionally having at least one substituent in the 2-, 3- or 4-positions, in addition to a possible substitutent or substituents at positions 6 to 9 if a substituted aniline of formula (IV), (IX) or (XII) is reacted with an iodoacid (XVIII).

wherein $R_6$ is as defined above can be converted to compounds of formula (VIII) where $R_1$ is as defined for formula (I) but additionally can represent COOH at positions 2-4 and 6-9.

Compounds of general formula (VIII) can be activated by reaction with a suitable halogen reagent (e.g. $PCl_5$, $POCl_3$, but preferably $SOCl_2$) to provide compounds of formula (II) where $R_5$ is Cl.

Similar activation of compounds of general formula (VIII) with $POBr_3$ or preferably $SOBr_2$ provides compounds of formula (II) where $R_5$ is Br.

Reaction of the phenzazine-1-carboxylic acids (VIII) with tris(4-nitrophenyl)phosphite in pyridine gives the 4-nitrophenylester derivatives (II) where $R_5$ is $OC_6H_5$-p-$NO_2$ (B. F. Cain, G. J. Atwell and W. A. Denny, J. Med. Chem., 20 987 (1977)).

Reaction of the phenazine-1-carboxylic acid (VIII) with 1,1'-carbonyldiimidazole in DMF or N-methylpyrrolidone gives the imidazolide derivatives of formula (II) where $R_5$ is 1-N-imidazole, i.e.

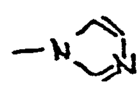

Reaction of the phenazine-1-carboxylic acid (VIII) with 2-chloro-N-methylpyridinium salts in DMF or N-methylpyrrolidone gives the 2-N-methylpyridinium salt esters of formula (II) where $R_5$ is a O-(2-N-methylpyridinium) salt, i.e.

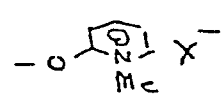

Reaction of the phenazine-1-carboxylic acid (VIII) with bis(1-N-benzotriazole)carbonate in DMF or N-methylpyrrolidone gives the 1-N-benzotriazole esters of formula (II) where $R_5$ is O-(1-N-benzotriazole), i.e.

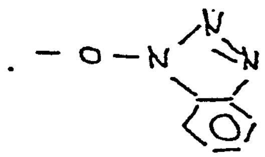

In the case of compounds of formula (VIII) where $R_1$ additionally can represent COOH at positions 2-4 and 6-9 the above described activation procedures provide compounds of formula (II) where $R_1$ additionally can represent $COR_5'$ at positions 2-4 and 6-9 and $R_5'$ is as defined above but dependent upon the activation procedure employed.

The compounds of general formula (II) where $R_5$ (and optionally $R_5'$) are as defined above are then coupled with suitable primary amines of general formula (III) as described above to provide compounds of general formula (I).

The N-oxide variants of formula (I) can be prepared for example by the general method outlined in Scheme V (<u>Chem. Abstr.</u> <u>74</u>:p88049).

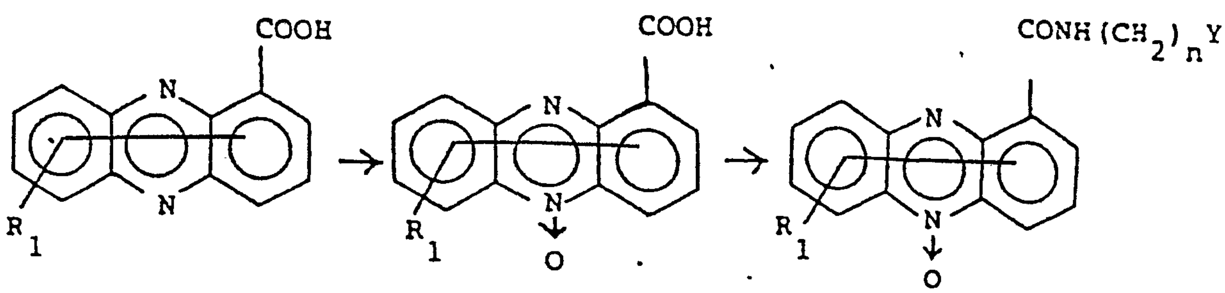

(VIII) (XX) (XXI)

SCHEME V

In Scheme V, $R_1$ is as defined above when in positions 2,3,7,8 and 9, but in position 4 and 6 is restricted to groups smaller than chloro.

Oxidation of phenazine-1-carboxylic acids (VIII) with $H_2O_2$ in acetic acid selectively provides the 5-N-oxide derivatives (IX) which can be elaborated by previously described methods to the desired products (X).

As will be appreciated, free bases/acids shown in the specification may, if appropriate, be in the form of salts, and compounds may be used in the form of their N-oxides or di-N-oxides if appropriate.

The following Tables I and II set out physical data for 28 compounds within the general formula (I), representative of it, and preparable by the processes of the invention. In Table I the following terms and abbreviations are used:-

| | | |
|---|---|---|
| MP | = | melting point of the reported acid addition salt in $^{o}C$. |
| Rm | = | a measure of the compound's lipophilic-hydrophilic balance from reversed phase partition chromatography. Rm is linearly related to partition co-efficients obtained in the 1-octanol/water system. |

TABLE I:

| No. | $R_1$ | n | Y | M.P. (°C) | Rm |
|---|---|---|---|---|---|
| 1 | H | 2 | $N(Me)_2$ | 232-237 | -0.29 |
| 2 | H | 3 | $N(Me)_2$ | 226-228 | -0.21 |
| 3 | H | 4 | $N(Me)_2$ | 186-191 | -0.11 |
| 4 | H | 2 | $N(Et)_2$ | 236-238 | 0.09 |
| 5 | H | 2 | $NH(CH_2)_2OH$ | 203-205 | (-0.27) |
| 6 | H;5N→O | 2 | $N(Me)_2$ | 215-217 | (-0.59) |
| 7 | $6-CONH(CH_2)_2N(Me)_2$ | 2 | $N(Me)_2$ | 270-271 | (-1.42) |
| 8 | $3-CH_3$ | 2 | $N(CH_3)_2$ | 236-238 | |
| 9 | $3-OCH_3$ | 2 | $N(CH_3)_2$ | 242-245(dec) | -0.25 |
| 10 | 3-Cl | 2 | $N(CH_3)_2$ | 250-253(dec) | -0.06 |
| 11 | $6-CH_3$ | 2 | $N(CH_3)_2$ | 246-248 | (0.0) |
| 12 | 6-Cl | 2 | $N(CH_3)_2$ | 257-260(dec) | -0.18 |
| 13 | $7-CH_3$ | 2 | $N(CH_3)_2$ | 225-227 | (-0.17) |
| 14 | 7-Cl | 2 | $N(CH_3)_2$ | 223-225 | (-0.14) |
| 15 | $8-OCH_3$ | 2 | $N(CH_3)_2$ | 229-231 | |
| 16 | 8-Cl | 2 | $N(CH_3)_2$ | 253-255 | |
| 17 | $9-CH_3$ | 2 | $N(CH_3)_2$ | 248-250 | -0.23 |
| 18 | $9-OCH_3$ | 2 | $N(CH_3)_2$ | 255(dec) | -0.41 |
| 19 | 9-Cl | 2 | $N(CH_3)_2$ | 246-249 | |
| 20 | $9-OCH_2CH_3$ | 2 | $N(CH_3)_2$ | 245-247 | -0.30 |

TABLE I: continued

| No. | $R_1$ | n | Y | M.P.(°C) | Rm |
|---|---|---|---|---|---|
| 21 | 9-OPh | 2 | $N(CH_3)_2$ | 239-241 | 0.04 |
| 22 | 6,9-di$OCH_3$ | 2 | $N(CH_3)_2$ | 250(dec) | -0.51 |
| 23 | 3,4-benz | 2 | $N(CH_3)_2$ | 254-256 | 0.11 |
| 24 | 6,7-benz | 2 | $N(CH_3)_2$ | 238-240 | -0.32 |
| 25 | 8,9-benz | 2 | $N(CH_3)_2$ | 227-230 | (-0.03) |

<u>TABLE II:</u> Elemental Analyses for the Compounds of Table I

| | | | Found | | | | Calculated | | | |
|---|---|---|---|---|---|---|---|---|---|---|
| | | MW | C | H | N | Cl | C | H | N | Cl |
| 1 | $C_{17}H_{18}N_4O.HCl$ | 330.8 | 61.9 | 6.0 | 17.1 | 11.0 | 61.7 | 5.8 | 16.9 | 10.7 |
| 2 | $C_{18}H_{20}N_4O.HCl$ | 344.8 | 62.6 | 6.1 | 16.0 | 10.5 | 62.7 | 6.1 | 16.3 | 10.3 |
| 3 | $C_{19}H_{22}N_4O.HCl$ | 358.9 | 63.6 | 6.7 | 15.7 | 10.2 | 63.6 | 6.5 | 15.6 | 9.9 |
| 4 | $C_{19}H_{22}N_4O.HCl$ | 358.9 | 63.3 | 6.5 | 15.6 | 10.0 | 63.6 | 6.5 | 15.6 | 9.9 |
| 5 | $C_{17}H_{18}N_4O_2.HCl$ | 346.8 | 58.4 | 5.1 | 16.1 | 10.3 | 58.9 | 5.5 | 16.2 | 10.2 |
| 6 | $C_{17}H_{18}N_4O_2.HCl.{}^1/_4H_2O$ | 351.3 | 57.9 | 5.2 | 15.8 | 10.5 | 58.1 | 5.6 | 16.0 | 10.1 |
| 7 | $C_{22}H_{28}N_6O_2.2HCl$ | 481.4 | 54.8 | 6.2 | 17.5 | 14.8 | 54.9 | 6.3 | 17.5 | 14.7 |
| 8 | $C_{18}H_{20}N_4O.HCl$ | 344.8 | 62.6 | 6.0 | 16.2 | 10.3 | 62.7 | 6.1 | 16.3 | 10.3 |
| 9 | $C_{18}H_{20}N_4O_2.HCl$ | 360.8 | 59.9 | 6.0 | 15.6 | 10.0 | 59.9 | 5.9 | 15.5 | 9.8 |
| 10 | $C_{17}H_{17}N_4O.HCl$ | 365.3 | 55.9 | 5.1 | 15.3 | 19.6 | 55.9 | 5.0 | 15.3 | 19.4 |
| 11 | $C_{18}H_{20}N_4O.HCl$ | 344.8 | 63.2 | 6.4 | 16.1 | 10.6 | 62.7 | 6.1 | 16.3 | 10.3 |
| 12 | $C_{17}H_{17}ClN_4O.HCl$ | 365.3 | 55.7 | 5.1 | 15.5 | 19.3 | 55.9 | 5.0 | 15.3 | 19.4 |
| 13 | $C_{18}H_{20}N_4O.HCl$ | 344.8 | 62.8 | 5.7 | 16.0 | 10.5 | 62.7 | 6.1 | 16.3 | 10.3 |
| 14 | $C_{17}H_{17}ClN_4O.HCl$ | 365.3 | 55.8 | 5.1 | 15.1 | 19.3 | 55.9 | 5.0 | 15.3 | 19.4 |
| 15 | $C_{18}H_{20}N_4O_2.HCl.{}^1/_2H_2O$ | 369.8 | 58.3 | 5.4 | 15.2 | | 58.5 | 6.0 | 15.2 | |
| 16 | $C_{17}H_{17}ClN_4O.HCl$ | 365.3 | 55.5 | 4.9 | 15.2 | 19.4 | 55.9 | 5.0 | 15.3 | 19.4 |
| 17 | $C_{18}H_{20}N_4O.HCl$ | 344.8 | 62.7 | 6.0 | 16.1 | 10.6 | 62.7 | 6.1 | 16.3 | 10.3 |
| 18 | $C_{18}H_{20}N_4O_2.HCl$ | 360.8 | 59.7 | 5.8 | 15.3 | | 59.9 | 5.9 | 15.5 | |
| 19 | $C_{17}H_{17}ClN_4O.HCl$ | 365.3 | 55.6 | 4.7 | 15.2 | 19.6 | 55.9 | 5.0 | 15.3 | 19.4 |
| 20 | $C_{19}H_{22}N_4O_2.HCl$ | 374.9 | 60.8 | 6.1 | 14.8 | 9.8 | 60.9 | 6.2 | 14.9 | 9.5 |
| 21 | $C_{23}H_{22}N_4O_2.HCl$ | 422.9 | 65.2 | 5.3 | 13.2 | 8.6 | 65.3 | 5.5 | 13.3 | 8.4 |
| 22 | $C_{19}H_{22}N_4O_3.HCl$ | 390.9 | 57.9 | 5.4 | 14.2 | 9.6 | 58.4 | 5.9 | 14.3 | 9.1 |
| 23 | $C_{21}H_{20}N_4O.HCl$ | 380.9 | 66.2 | 5.6 | 14.7 | 9.4 | 66.2 | 5.6 | 14.7 | 9.3 |
| 24 | $C_{21}H_{20}N_4O.HCl$ | 380.9 | 66.2 | 5.9 | 14.9 | 9.3 | 66.2 | 5.6 | 14.7 | 9.3 |
| 25 | $C_{21}H_{20}N_4O.HCl$ | 380.9 | 66.5 | 5.3 | 14.5 | 9.6 | 66.2 | 5.6 | 14.7 | 9.3 |

The following Examples illustrate the preparation of compounds of the general formula (I)

Example A: Preparation of Compound 1 of Table I by the Method of Scheme I

N-Phenyl-3-nitroanthranilic acid (VI; $R_6$=H)

A mixture of 20g (0.081mol) of 2-bromo-3-nitrobenzoic acid, 15g (0.16mol) aniline, 1g CuCl, 25ml N-ethylmorpholine and 40ml butane-2,3-diol was heated at 80°C for 4h and the cooled mixture was diluted with 0.1N aqueous $NH_3$ (200ml). After being clarified with activated charcoal and filtered through celite the deep red solution was poured slowly into dilute HCl to give N-phenyl-3-nitroanthranilic acid, (19.4g, 74% yield) M.P. (MeOH(aq)) 192-193°C (lit 195-196°C. - A.A. Goldberg and W. Kelly, JCS 1946,102.)

Phenazine-1-carboxylic acid (VII; $R_6$=H = VIII; $R_1$=H)

A solution of 3.30g (0.013mole) of N-phenyl-3-nitroanthranilic acid in 75ml absolute ethanol containing 3g dissolved sodium was treated with 2g $NaBH_4$ and the mixture was heated under reflux for 12h. The majority of the solvent was boiled off and the residue was dissolved in water. The filtered solution was then poured into dilute HCl to give phenazine-1-carboxylic acid (2.21g, 77% yield) M.P. (MeOH) 240-241°C (lit 239-240°C. - S.R. Challand, R.B. Herbert and F.G. Holliman, J.C.S. Chem.Comm. 1970, 1423).

N-(2-Dimethylaminoethyl)phenazine-1-carboxamide hydrochloride (Compound 1 of Table I)

A solution of 1g (4.5mmol) phenazine-1-carboxylic acid in 50ml $SOCl_2$ was refluxed for 30min and the excess $SOCl_2$ was removed under vacuum. A solution of 3ml of N,N-dimethylethylenediamine in dry $CH_2Cl_2$ (200ml) was added and the mixture was stirred to dissolve all of the solid. The $CH_2Cl_2$ solution was washed twice with water and extracted twice with dilute HCl. The aqueous layer was basified with conc. $NH_3$ solution and extracted with chloroform. Drying and removal of the solvent gave a solid which was dissolved in the minimum volume of methanol and treated with 1 equivalent of conc. HCl. Addition of ethyl acetate gave a crystalline precipitate which was recrystallized from methanol- ethyl acetate to give N-(2-dimethylaminoethyl)- phenazine-1-carboxamide hydrochloride (compound 1 of Table I) (0.97g, 66% yield).

Compounds 2,3 and 4 of Table I were similarly prepared from phenazine-1-carboxylic acid by substitution of the appropriate amine in the above procedure.

Example B: Preparation of Compound 21 of Table I by the Method of Scheme I.

N-(2-Phenoxyphenyl)-3-nitroanthranilic acid (VI; $R_6$=3' - OPh)

A mixture of 15g (0.08mol) 2-aminodiphenyl ether, 10g (0.04mol) 2-bromo-3-nitrobenzoic acid, 1g CuCl, 15ml N-ethylmorpholine and 25ml butane-2,3-diol was stirred at 80°C for 4h and allowed to cool. The mixture was diluted with 0.01N NaOH solution, clarified with activated charcoal and filtered through celite. The cloudy solution was then seeded with 2-aminodiphenyl ether to precipitate unreacted starting material. The resulting clear red coloured solution was acidified with

dilute HCl to give N-(2-phenoxyphenyl)-3-nitroanthranilic acid, 13.0g, 91% yield, M.P. (benzene-pet. ether) 153-155°C. Anal. Found C, 64.9; H, 3.9; N, 8.0; $C_{19}H_{14}N_2O_5$ requires C, 61.1; H, 4.0; N, 8.0%.

9-Phenoxyphenazine-1-carboxylic acid (VII; $R_6$=9-OPh = VIII; $R_1$=9-OPh)

N-(2-Phenoxyphenyl)-3-nitroanthranilic acid (10g, 2.85 mmol) was refluxed with 10g $NaBH_4$ in 600ml 2M NaOH for 48h. The cooled mixture was filtered and the crude product collected. This was dissolved in hot water and acidified with conc. HCl to give a precipitate which consisted of a mixture of the desired product and the amine derived by direct reduction of the starting material. The dried mixture was suspended in boiling methanol, sufficient triethylamine was added to ensure all solid dissolved, and after being filtered the solution was acidified with glacial acetic acid to give crystals of 9-phenoxyphenazine-1-carboxylic acid (yield 0.93g, 10%), M.P. 232-234°. (Analysis: C,72.1; H,3.9; N,8.9. $C_{19}H_{12}N_2O_3$ requires C,72.1; H,3.8; N,8.9%).

The filtrate from the original reaction mixture was acidifed with conc. HCl to give phenazine-1-carboxylic acid (yield 1.58g, 25%), formed as a result of displacement of the phenoxy group during ring closure.

N-(2-Dimethylaminoethyl)-9-phenoxyphenazine-1-carboxamide, hydrochloride. Compound 21 of Table I.

A solution of 9-phenoxyphenazine-1-carboxylic acid (1g, 2.85mmol) in 30 ml $SOCl_2$ was refluxed for 30min, and after removal of the solvent under vacuum, the residue was treated with a solution of N,N-dimethylethylenediamine (3ml) in $CH_2Cl_2$(200ml). The $CH_2Cl_2$

solution was washed twice with water and extracted four times with 0.1M HCl. The aqueous layer was basified with conc. $NH_3$ solution and extracted with fresh $CH_2Cl_2$. Drying and removal of the solvent gave 0.8g (73%) of N-(2-dimethylaminoethyl)-9-phenoxyphenazine-1- carboxamide which was converted to its hydrochloride salt by treating a methanolic solution with 1 equivalent of conc. HCl. The product was isolated by precipitation with ethyl acetate, and recrystallized from methanol-ethyl acetate. M.P. 239-241°C.

Example C: Preparation of Compound 18 of Table I by the Method of Scheme II

N-(2,6-dimethoxyphenyl)-3-nitroanthranilic acid (X; $R_6$ = $OCH_3$)

A mixture of 6.85g (0.05mol) 2,6-dimethoxyaniline, 12.4g (0.05mol) 2-bromo-3-nitrobenzoic acid, 1g CuCl, 20ml N-ethylmorpholine and 25ml butane-2,3-diol was stirred and heated at 80°C under nitrogen for 3h. After cooling the mixture was diluted with 0.01N $NH_3$ (aq) solution, activated charcoal was added and the solution was filtered through celite. Acidification with dilute HCl gave N-(2,6-dimethoxyphenyl)-3-nitroanthranilic acid (6.41g, 40% yield). M.P. (benzene) 176-178°C. Anal. Found C, 56.2; H, 4.4; N, 8.7; $C_{15}H_{14}N_2O_6$ requires C, 56.6; H, 4.4; N, 8.9%.

9-Methoxyphenazine-1-carboxylic acid (VIIA; $R_6$=$OCH_3$ = VIII; $R_1$=9-$OCH_3$)

To a solution of the nitroanthranilic acid (15.4g, 0.048mol) in 500ml MeOH containing 23g dissolved sodium was added 10g $NaBH_4$ and the mixture was refluxed for 48h. The solvent was removed under vacuum and

the residue was dissolved in water and filtered. Acidification with dilute HCl gave the crude product which was dried and extracted with chloroform. Removal of the chloroform gave 9-methoxyphenazine-1-carboxylic acid (7.1g, 58% yield) M.P. (MeOH) 258-259°C (lit 262-263°C - P.K. Brooke, S. R. Challand, M. E. Flood, R. B. Herbert, F. G. Holliman and P. N. Ibberson, J. C. S. Perkin I, 1976, 2248). (When the above reduction was performed in 2N NaOH the isolated yield was 17%).

N-(2-Dimethylaminoethyl)-9-methoxyphenazine-1-carboxamide hydrochloride (Compound 18 of Table I)

A suspension of 5g (0.02mol) 9-methoxyphenazine-1-carboxylic acid in 55ml DMF was treated successively with 6.5g 1,1'-carbonyldiimidazole and 6ml N,N-dimethylethylenediamine to give, after treatment with 1 equivalent of conc. hydrochloric acid, N-(2-dimethylaminoethyl)-9-methoxyphenazine-1-carboxamide (6g, 83%) M.P. (MeOH-EtOAc) 255°C (dec).

Example D: Preparation of Compound 16 of Table I by the Method of Scheme III

N-(5-Chloro-2-fluorophyenyl)-3-nitroanthranilic acid (XIII; $R_6$=5'-Cl)

A mixture of 17g (0.117mol) 5-chloro-2-fluoroaniline, 19g (0.077mol) 2-bromo-3-nitrobenzoic acid, 2g CuCl, 30ml N-ethylmorpholine and 40ml butane-2,3-diol was heated and stirred at 90°C for 20h. Standard workup gave N-(5-chloro-2-fluorophenyl)-3-nitroanthranilic acid (7.5g, 31% yield), M.P. (benzene) 199-201°C. Anal. Found C, 50.5; H, 2.4; N, 8.8; $C_{13}H_8ClF\ N_2O_4$ requires C, 50.3; H, 2.6; N, 9.0%.

8-Chlorophenazine-1-carboxylic acid (VII; $R_6$=8-Cl = VIII; $R_1$=8-Cl)

A mixture of 6.8g (0.022mol) of the nitroanthranilic acid and 4g $NaBH_4$ in 550ml 2N NaOH was refluxed for 1h and cooled. The crystalline precipitate was collected, washed with 2N NaOH, dissolved in water, and the solution was filtered.

Acidification with dil. HCl gave 8-chlorophenazine-1-carboxylic acid (3.62g, 64% yield) M.P. (MeOH) 286-289°C. Anal. Found C, 60.0; H, 2.7; N, 11.0; Cl, 14.0; $C_{13}H_7ClN_2O_2$ requires C, 60.4; H, 2.7; N, 10.8; Cl 13.7%.

N-(2-Dimethylaminoethyl)-8-chlorophenazine-1-carboxamide hydrochloride (Compound 16 of Table I)

A suspension of 1.42g (0.0055mol) 8-chlorophenazine-1-carboxylic acid in 40ml DMF was treated successively with 1,1'-carbonyldiimidazole (1.8g, 0.01mol) and 5ml N,N-dimethylaminoethylenediamine in 100ml $CH_2Cl_2$. The organic layer was washed (2x) with water and extracted with dil. HCl. Basification with dil. $NH_3$ (aq) and extraction with $CHCl_3$ gave N-(2-dimethylaminoethyl)-8-chlorophenazine-1-carboxamide which was converted to its hydrochloride salt and recrystallized (overall yield 1.5 g, 75%). M.P. (MeOH-EtOAc) 253-255°C.

Compound 5 of Table I was similarly prepared from phenazine-1-carboxylic acid (prepared according to Example A) and N-(2-hydroxyethyl)ethylenediamine by using 1,1'- carbonyldiimidazole as the coupling reagent, using the procedure described in Example D.

0172744

## Example E: Preparation of Compound 8 of Table I by the Method of Scheme IV

3-Methylphenazine-1-carboxylic acid (VIIB: $R_6$=3-$CH_3$ = VIII; $R_1$=3-$CH_3$)

Reaction of 4-methyl-2-nitroaniline with chloral and hydroxylamine under the standard conditions (C.S. Marvel and G.S. Hiers, "Organic Synthesis", Coll. Vol. 1, 1941 P.327) gave 4-methyl-2-nitroisonitrosoacetanilide (XV, $R_6$ = 4-$CH_3$) in 33% yield. M.P. (pet. ether) 153-156°C. Anal. Found C, 48.7; H, 4.1; N, 18.8; $C_9H_9N_3O_4$ requires C, 48.4; H, 4.1; N, 18.8%.

Ring closure of the isonitrosoacetanilide with conc. $H_2SO_4$ at 80-95°C for 15min gave 5-methyl-7-nitroisatin (XVI; $R_6$ = 5-$CH_3$) in 87% yield, M.P. (MeOH (aq)) 216-218°C (lit M.P. 202-203°C - Buu-Hoi Ng.Ph., Bull.Soc.chim France, 1946, 586).

A suspension of 15g (0.073mol) of the nitroisatin in 400ml 2N NaOH was treated with 15ml 27% $H_2O_2$ at room temperature and the mixture was stirred for 10min. The resulting solution was filtered and acidified with glacial acetic acid to give 5-methyl-3-nitroanthranilic acid, (XVII; $R_6$ = 5-$CH_3$) (13.57g, 95%) M.P. (MeOH) 253-255°C, (lit M.P. 256-257°C. - R. Adams and H.R. Snyder, J. Amer. Chem. Soc., 1938, 60, 1411).

A solution of 13.57g (0.069mol) of the anthranilic acid in 60ml conc. $H_2SO_4$ was combined with a solution of 7g (0.1mol) $NaNO_2$ in 60ml conc. $H_2SO_4$ at 0°C and to the stirred mixture was added 125ml 85% $H_3PO_4$ at a rate designed to keep the temperature below 10°C. After a further 3h 8g urea was added and 15min later the mixture was poured onto 500g ice. The filtered solution was treated with 25g KI in

40ml water and the mixture was heated in a boiling waterbath for 15min, and cooled. Filtration and drying gave 2-iodo-5- methyl-3-nitrobenzoic acid, (XVIII; $R_6$ = 5-$CH_3$) (19.26g, 91% yield), M.P. (MeOH (aq)) 210-212°C, (lit M.P. 207-209°C-R. Adams and H.R. Snyder, JACS, 1938, 60, 1411).

A mixture of 19.26g (0.063mol) of the iodo acid, 11.2g (0.12mol) aniline, 2g CuCl, 25ml N-ethylmorpholine and 40ml butane-2,3-diol was heated at 90°C for 3h and the product was extracted into dilute $NH_3$ (aq) solution. Filtration through celite and acidification gave N-phenyl-5-methyl-3-nitroanthranilic acid, (XIX; $R_6$ = 5-$CH_3$), (15.3g, 89% yield), M.P. (benzene) 177-179°C (lit M.P. 174°C - S.M. Sane, S.N. Chakravarty and B.N. Parmanick, J. Ind. Chem. Soc., 1932, 9, 55. Chem. Abstr, 1932, 26, 4037).

A solution of 12g of the nitroanthranilic acid in 500 ml EtOH containing 23g Na was treated with 6g $NaBH_4$ and the mixture was heated under reflux for 1h. Most of the solvent was removed under vacuum, and the residue was dissolved in water and filtered. Acidification with dilute HCl gave 3-methylphenazine-1-carboxylic acid (VIIB: $R_6$ =3-$CH_3$) (5.7g, 48%) M.P. (MeOH) 212-213°C. Anal. Found C, 70.5; H, 4.4; N, 11.6; $C_{14}H_{10}N_2O_2$ requires C, 70.5; H, 4.2; N, 11.8%.

N-(2-Dimethylaminoethyl)-3-methylphenazine-1-carboxamide hydrochloride (Compound 8 of Table I)

A stirred suspension of 2g (8.4mmol) of 3-methylphenazine-1-carboxylic acid in 40ml DMF was treated with 2.7g (0.017mol) 1,1'-carbonyldiimidazole at room temperature and a clear solution was obtained after 5min to be shortly followed by crystallization of the imidazolide. A solution of 5ml N,N-dimethylethylendiamine in 100ml

$CH_2Cl_2$ was added and the reaction mixture was worked up in the usual way to give N-(2-dimethylaminoethyl)-3-methylphenazine-1- carboxamide hydrochloride (2.25g 78%) M.P. (Me OH-EtOAc) 236-238°C.

Example F: Preparation of Compound 6 of Table I by the Method of Scheme V

Phenazine-1-carboxylic acid-5-N-oxide (XX; $R_1$=H)

A solution of phenazine-1-carboxylic acid (2.5g, 11.2mmol) in 250ml glacial acetic acid was diluted with 50ml 30% $H_2O_2$ and the resulting solution was stirred at 55-60°C for 24h before being diluted with 500ml of water and cooled. The orange-yellow crystals were collected and dried (yield 1.01g, 38%). M.P. (AcOH) 222-223°C (lit. M.P. 223°C - S. Watanabe, O. Shigeru, M. Sugiyama, H. Kondo, German Patent 1,935,705, 19 Mar 1970. Chem. Abstr. 1970, 72: 134173d).

N-(2-Dimethylaminoethyl)phenazine-1-carboxamide-5-N-oxide hydrochloride (Compound 6 of Table I).

A suspension of phenazine-1-carboxylic acid-5-N-oxide (1g, 4.16mmol) in 15ml dry DMF was treated with 1.4g (2 equivalents) of 1,1'-carbonyldiimidazole and a clear solution was obtained within 5 minutes. N,N-dimethylethylenediamine (2ml) was added to the cooled solution, and after a further 5min the mixture was diluted with 150ml of dichloromethane. The organic layer was washed twice with water, and then extracted with 0.1M hydrochloric acid. The aqueous layer was basified with conc. aqueous ammonia and extracted with dichloromethane. After being dried ($Na_2SO_4$), the solvent was removed and the residue

0172744

was dissolved in the minimum volume of methanol. Conc. hydrochloric acid (0.5ml) was added and the solution was diluted with ethyl acetate to give the product which was collected, washed with acetone, and recrystallized from methanol-ethyl acetate. (Yield 0.98g, 68%. M.P. 215-217°C.

Example G

The substituted phenazine carboxylic acids for the preparation of compounds 7,9-15,17,19,20 and 22-25 of Table I were prepared essentially by the methods described in the preceding Examples A to E; brief details for the preparation of each are given immediately below. The substituted phenazine carboxylic acid were each then reacted with N,N-dimethylethylenediamine either by preparation of the acid chloride using the procedure described in Example A, or by coupling of the acid using 1,1'-carbonyldiimidazole using the procedure described in Example D.

Phenazine-1,6-dicarboxylic acid (VIII; $R_1$=6-$CO_2H$) was prepared by reduction of 2-(3-carboxyphenyl)-3-nitro anthranilic acid (VI; $R_6$=3-$CO_2H$) in ethanol (M.E. Flood, R.B. Herbert and F.G. Holliman, J.C.S. Perkin I 1972, 622.). The small amount of 1,8-diacid was removed by recrystallization from DMF.

3-Methoxyphenazine-1-carboxylic acid (VIII; $R_1$=3-$OCH_3$) was prepared from 3-chlorophenazine-1-carboxylic acid (VII; $R_6$=3-Cl) by displacement of the halogen with methoxide in methanol.

3-Chlorophenazine-1-carboxylic acid (VIII; $R_1$=3-Cl) was prepared from N-phenyl-5-chloro-3-nitroanthranilic acid (VI; $R_6$=5-Cl) by reduction in water. The latter compound was formed by reaction of aniline with 2,5-dichloro-3-nitrobenzoic acid.

6-Methylphenazine-1-carboxylic acid (VIII; $R_1$=6-$CH_3$) was prepared by reduction of N-(3-methylphenyl)-3-nitroanthranilic acid (VI; $R_6$=3'-$CH_3$) in ethanol. It was separated from the isomeric 8-methyl acid by chromatography of the methyl esters on $SiO_2$ (H. Budzikiewicz, D. Stockl and A. Romer, J. Heterocyclic Chem., 1979, 16, 1307.).

6-Chlorophenazine-1-carboxylic acid (VIII; $R_1$=6-Cl) was prepared by reduction of N-(3-chlorophenyl)-3-nitroanthranilic acid (VI; $R_6$=3'-Cl) in water. It was separated from the isomeric 8-chloro acid by recrystallization from DMF.

7-Methylphenazine-1-carboxylic acid (VIII; $R_1$=7-$CH_3$), 9-Methylphenazine-1-carboxylic acid (VIII; $R_1$=9-$CH_3$) benzo[a]phenazine-8-carboxylic acid (VIII; $R_1$=6,7-benz) and benzo[a]phenazine-11-carboxylic acid (VIII; $R_1$=8,9-benz) were prepared by reduction of N-(4-methylphenyl)-3-nitroanthranilic acid (VI; $R_6$=4-$CH_3$), N-(2-methylphenyl)-3-nitroanthranilic acid (VI; $R_6$=2-$CH_3$), N-(2-naphthyl)-3-nitroanthranilic acid (VI; $R_6$=3,4-benz) and N-(1-naphthyl)-3-nitroanthranilic acid (VI; $R_6$=2,3-benz) respectively in ethanol.

6,9-dimethoxyphenazine-1-carboxylic acid [VIII; $R_1$=6,9-di($OCH_3$)] was prepared by reduction of N-(2,5-dimethoxyphenyl)-3-nitroanthranilic acid [VI; $R_6$=2,5-di($OCH_3$] in methanol.

7-Chlorophenazine-1-carboxylic acid (VIII; $R_1$=7-Cl) and 9-chlorophenazine-1-carboxylic acid (VIII; $R_1$=9-Cl) were prepared by reduction of N-(4-chlorophenyl)-3-nitroanthranilic acid (VI; $R_6$=4-Cl) and N-(2-chlorophenyl)-3-nitroanthranilic acid (VI; $R_6$=2-Cl) respectively in water. The 9-chloro acid was separated from by-product phenazine-1-carboxylic acid by recrystallization from DMF.

8-Methoxyphenazine-1-carboxylic acid (VIII; $R_1$=8-$OCH_3$) was prepared from 8-chlorophenazine-1-carboxylic acid (VII; $R_6$=8-Cl) by displacement of the halogen with methoxide in methanol.

9-Ethoxyphenazin-1-carboxylic acid (VIII; $R_1$=9-$OCH_2CH_3$) was prepared from N-(2-ethoxyphenyl)-3-nitroanthranilic acid by reduction in ethanol. By-product phenazine-1-carboxylic acid was removed by recrystallization from DMF.

Benzo[a]phenazine-6-carboxylic acid (VIII; $R_1$=3,4-benz) was prepared by sodium dithionite reduction of benzo[a]phenazine-6-carboxylic acid-7,12-di-N-oxide which was obtained from reaction of benzofuroxan with 3-hydroxy-2-naphthoic acid (K. Ley and F. Seng, Synthesis 1975, 415).

Physical data for the preceding substituted phenazine carboxylic acids are set out in the following Table :

| Compound VIII for preparing Compound No. | $R_6$ | M.P.(°C) | Lit.M.P. (°C) / Formula | | Analysis C | H | N | Cl |
|---|---|---|---|---|---|---|---|---|
| 9 | 3-$OCH_3$ | 242-243 | $C_{14}H_{10}N_2O_3$ | Found<br>Calc | 66.1<br>66.1 | 3.9<br>4.0 | 11.0<br>11.0 | |
| 10 | 3-Cl | 255-257 | $C_{13}H_7ClN_2O_2$ | Found<br>Calc | 60.2<br>60.4 | 2.7<br>2.7 | 10.8<br>10.8 | 13.9<br>13.7 |
| 11 | 6-$CH_3$ | 216-217 | $C_{14}H_{10}N_2O_2$ | Found<br>Calc | 70.4<br>70.6 | 4.2<br>4.2 | 11.6<br>11.8 | |
| 12 | 6-Cl | 310 dec | $C_{13}H_7ClN_2O_2$ | Found<br>Calc | 60.1<br>60.4 | 2.7<br>2.7 | 10.9<br>10.8 | 13.9<br>13.7 |
| 13 | 7-$CH_3$ | 260 dec | $C_{14}H_{10}N_2O_2$ | Found<br>Calc | 70.7<br>70.6 | 4.1<br>4.2 | 11.6<br>11.8 | |
| 14 | 7-Cl | 274 dec | $C_{13}H_7ClN_2O_2$ | Found<br>Calc | 60.6<br>60.4 | 2.5<br>2.7 | 10.7<br>10.8 | 14.0<br>13.7 |
| 19 | 9-Cl | 272-273 | $C_{13}H_7ClN_2O_2$ | Found<br>Calc | 60.6<br>60.4 | 2.9<br>2.7 | 10.6<br>10.8 | 13.8<br>13.7 |
| 7 | 6-$CO_2H$ | 290 dec | 287-290 - M.E. Flood, R.B. Herbert and F.G. Holliman J.C.S. Perkin 1, 1972, 622 | | | | | |
| 15 | 8-$OCH_3$ | 286-288 | 292-293 - P.K. Brooke, S.R. Challand, M.E. Flood, R.B. Herbert and F.G. Holliman, J.C.S. Perkin I, 1976, 2248 | | | | | |
| 17 | 9-$CH_3$ | 237-238 | 235 - E Breitmaier and U. Hollstein, J.Org. Chem., 1976, 41, 2104. | | | | | |
| 20 | 9-$OCH_2CH_3$ | 237-239 | 230-231.5 - P.K. Brooke, S.R. Challand, M.E. Flood, R.B. Herbert and F. G. Holliman, J.C.S. Perkin I, 1976, 2248. | | | | | |
| 22 | 6,9-di($OCH_3$) | 282-285 | 277 - Y.S. Rozum, Ukrain. Khim. Zhur., 1955, 21, 361. Chem.Abs., 1955, 49: 14772C. | | | | | |
| 23 | 3,4-benz | 272-274 | 275-278 - F. Uhllmann and R. Heisler, Ber., 1909, 42, 4263. | | | | | |
| 24 | 6,7-benz | 255-256 | 255 - R. Huisgen and G. Sorge, Ann., 1950, 566, 162. | | | | | |
| 25 | 8,9-benz | 243-246 | 256 - L. Birkofer and A. Widmann, Chem. Ber., 1953, 86, 1295 | | | | | |

The compounds of general formula (I), and particularly compounds listed in Tables I and II have antitumour activity in both *in vitro* and in *in vivo* test systems, as shown by the data of Table III.

The following Table III gives biological data for compounds whose physical data has been given in Tables I and II. The abbreviations used in Table III are:-

no. The number given to the corresponding compound in Table I.

$IC_{50}$ The nanomolar concentration of drug which when added to cultures of L1210 leukemia cells for 70h reduces counted cell numbers to 50% of controls. $IC_{50}$ values lower than 2500nM are considered significant.

OD The optimal drug dose (in milligrams per kilogram), administered as a solution in 0.1ml of 30% v/v ethyl alcohol in water on days 1, 5 and 9 after tumour inoculation. The drug is administered as a soluble acid addition salt.

ILSmax. The percentage increase in lifespan of treated animals over that of control animals injected with tumour alone. The average survival of control mice was 11 days (for P388 leukemia) and 17 days (for Lewis lung carcinoma). ILS values greater than 20% (P388) and 40% (Lewis lung) are considered statistically significant. Numbers in parentheses after ILS values indicate the number of long-term survivors (out of a group of 6).

Y Implies a significant value of drug activity at the stated dose.

N Implies no or not statistically significant activity at the stated dose.

P388 Is the P388 leukemia.

LL is the Lewis lung carcinoma.

Both of these tumour lines were obtained as frozen cell stocks from Mason Research Inc., U.S.A., and are passaged intraperitoneally in DBA-2 mice of either sex (P388) or subcutaneously in C57-B1 mice of either sex (Lewis lung) according to the standard methods (Cancer Chemother. Reports, 3, Part 3, p.9, 1972).

Groups of six mice (F1 hybrids of DBA-2 male x C57-B1 female) of weight 20 ± 1g were injected intraperitoneally (P388) or intravenously (tail vein, Lewis lung) with $10^6$ tumour cells on day 0. When given in this manner, P388 cells grow diffusely in the peritoneal cavity, whereas the Lewis lung cells form distinct solid tumour nodules in the lungs.

**TABLE III:** Biological Activity for the Compounds of Table I

| | L1210 | P388 in vivo | | | LL in vivo | | |
|---|---|---|---|---|---|---|---|
| No. | (IC50) | OD | ILS | Active | OD | ILS | Active |
| 1 | 1,400 | 150 | 88 | Y | 150 | 57 | Y |
| 2 | 1,500 | 65 | <20 | N | 225 | <40 | N |
| 3 | 1,171 | 100 | 49 | Y | 225 | <40 | N |
| 4 | 2,100 | 225 | 43 | Y | 150 | <40 | N |
| 5 | 2,000 | 65 | <20 | N | 65 | <40 | N |
| 6 | 3,000 | 150 | 80 | Y | 150 | 51 | Y |
| 7 | 210 | 100 | – | N | 100 | <40 | N |
| 8 | 410 | 150 | – | Y | – | – | – |
| 9 | 200 | 100 | 22 | Y | 150 | <40 | N |
| 10 | 156 | 100 | 73 | Y | 150 | 63 | Y |
| 11 | 820 | 150 | 58 | Y | 150 | <40 | N |
| 12 | 780 | 150 | 56 | Y | 150 | 49 | Y |
| 13 | 1,500 | 150 | 29 | Y | 100 | <40 | N |
| 14 | 1,140 | 65 | 31 | Y | 65 | <40 | N |
| 15 | 1,050 | 100 | 39 | Y | 150 | <40 | N |
| 16 | 680 | 100 | 64 | Y | 100 | <40 | N |
| 17 | 42 | 65 | 45 | Y | 65 | 62(1) | Y |
| 18 | 48 | 100 | 106 | Y | 225 | 128(5) | Y |
| 19 | 11 | 30 | – | Y | – | – | – |
| 20 | 100 | 100 | 92 | Y | 150 | 84(4) | Y |
| 21 | 1,900 | 65 | <20 | N | 65 | <40 | N |
| 22 | 1,630 | 150 | 26 | Y | 225 | <40 | N |
| 23 | 170 | 65 | 84 | Y | 65 | <40 | N |
| 24 | 310 | 45 | 29 | Y | 45 | <40 | N |
| 25 | 33 | 30 | 98 | Y | 45 | 56 | Y |

It is clear from the data of Table III that the phenazinecarboxamides of general formula (I) include compounds which are active antitumour agents, giving significant levels of life extension when tested against the P388 leukemia or Lewis lung carcinoma systems when given by intraperitoneal or intravenous injection respectively, and/or significant inhibition of cultured L1210 leukemia cells _in vitro_. The compounds also show antitumour activity when given by oral and intravenous routes. In addition to high cytotoxicity towards cultured L1210 leukemia cells, they are active in a number of other cultured tumour cell lines, including those originating from human breast and colon tumours.

These compounds of general formula (I) are thus indicated for use as antitumour agents.

The compounds also have antibacterial activity; specifically compounds 18 and 22 show _in vitro_ activity (MIC 1.000 ug/mL) against Escherichia coli 04863, Salmonella typhimurium TA1535, Alcaligenes viscolactis 21698, Branhamella catarrhalis 03596, Micrococcus luteus 05064, Staphylococcus aureus 02482, Streptococcus pyogenes C203, Streptococcus pneumoniae SV1, Streptococcus faecalis 05045, Bacillus cereus 04810, Bacillus megaterium 066 and Schizosaccharomyces pombe M1388. Compounds _1_ and 17 show activity against all of the above bacterial strains and, in addition, are active (MIC 1000 ug/mL) against Saccharomyces cerevisiae S288, Rhodotorula aurantiaca M1508, Torulopsis albida M1390 and Mucor parasiticus M2652. Thus, the invention provides for the use of these compounds as antibacterial agents.

The present invention therefore also provides a compound of formula (I) or a pharmaceutically acceptable acid addition salt, 5- or 10-mono-N-oxide or 5,10-di-N-oxide thereof, for use in the treatment of tumours, and in particular cancers.

The present invention further also provides pharmaceutical compositions having antitumour activity and comprising at least one compound of general formula (I) or a pharmaceutically acceptable acid addition salt, 5- or 10-mono-N-oxide or 5,10-di-N-oxide thereof, and one or more pharmaceutically acceptable carriers or diluents.

The active compounds may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of ingestible tablets, buccal tablets, troches, capsules, elixirs, suspensions, syrups, wafers and the like. Generally, such compositions and preparations should contain at least 0.1% of active compound. The percentage in the compositions and preparations may, of course, be varied and may conveniently be between about 2 and about 60% of the weight of the unit. The amount of active compound in such therapeutically useful compositions is such that a suitable dosage will be obtained. Preferred compositions or preparations according to the present invention are prepared so that an oral dosage unit form contains from about 5 to about 200 milligrams of active compound.

The tablets, troches, pills, capsules and the like may also contain, for example, one or more of the following: a binder such as gum tragacanth, acacia, corn starch or gelatin; excipients such as dicalcium phosphate; a distintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen or cherry flavouring. When the dosage

unit form is a capsule, it may contain, for example in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compounds may be incorporated into sustained-release preparations and formulations.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganims such as bacteria and fungi. The carrier can be a solvent or dispersion medium containing, for example, water, ethanol, polyol (for example, glycerol, propylene glycol, and

liquid polyethylene glycol, and the like), suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile- filtered solution thereof.

As used herein, "pharmaceutically acceptable carrier" includes any and all suitable solvents, dispersion media, coatings, antibacterial and antifungal agents, isotonic and absorption delaying agents and the like. The use of such media and agents for pharmaceutically active

substances is well-known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. "Dosage unit form" as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically-acceptable carrier in dosage unit form as hereinbefore disclosed. A unit dosage form can, for example, contain the principle active compound in amounts ranging from about 0.1 to about 400 mg, with from about one to about 30 mg being preferred. Expressed in proportions, the active compound is generally present in from about 0.1 to about 400 mg/ml of carrier. In the case of compositions containing supplementary active ingredients, the dosages are determined by reference to the usual dose and manner of administration of the said ingredients.

CLAIMS FOR ALL CONTRACTING STATES EXCEPT AUSTRIA

1. A compound represented by the general formula (I),

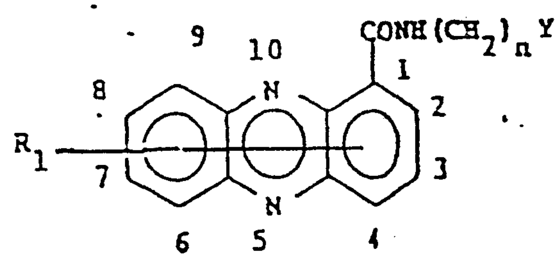

(I)

(I)

where $R_1$ represents H or up to three substituents, at positions selected from 2 to 4 and 6 to 9, wherein any two or all of the substituents may be the same or different and the substituents are selected from

lower alkyl radicals;

lower alkyl radicals substituted by one or more of the same or different substituents selected from hydroxy, amino and ether functions;

OH,

SH;

$OCH_2Ph$;

OPh;

$NO_2$;

halogen;

$CF_3$;

amino;

$NHSO_2R_2$, $NHCOR_2$, $NHCOOR_2$, $OR_2$ and $SR_2$ (where $R_2$ represents a lower alkyl radical which is unsubstituted or substituted by one or more of the same or different substituents selected from hydroxy, amino and ether

functions); and

$CONH(CH_2)_{n'}Y$ (where n' and Y' are as defined below), there being a maximum of one $CONH(CH_2)_{n'}Y'$ group;

or any two of $R_1$ at adjacent positions represent -CH=CH-CH=CH- as part of an extra benzene ring or $-O-CH_2-O-$ (methylenedioxy) and the third of $R_1$ has any one of the meanings given above with the exception of an OH at position 2;

Y and Y', which may be the same or different, each represents $C(NH)NH_2$, $NHC(NH)NH_2$ or $NR_3R_4$, where each of $R_3$ and $R_4$, which may be the same or different, represents H or a lower alkyl radical unsubstituted or substituted by one or more of the same or different substituents selected from hydroxy, amino and ether functions, or $R_3$ and $R_4$, together with the nitrogen atom to which they are attached, form a heterocyclic ring; and

n and n', which may be the same or different, each represents an integer from 2 to 6;

or an acid addition salt, 5- or 10- mono-N-oxide or 5,10-di-N-oxide thereof.

2. A compound according to claim 1 where $R_1$ represents one substituent selected from alkyl, unsubstituted or substituted by one or more of the same or different substituents selected from hydroxy, amino and ether functions; OH, SH, $OCH_2Ph$, OPh, $NO_2$, halogen, $CF_3$, $NH_2$, or $NHSO_2R_2$, $NHCOR_2$, $NHCOOR_2$, $OR_2$ or $SR_2$ (where $R_2$

represents a lower alkyl radical unsubstituted or substituted by one or more of the same or different substituents selected from hydroxy, amino and ether functions) at position 9; or where $R_1$ represents -CH=CH-CH=CH- at positions 8 and 9 as part of an extra benzene ring; Y represents $N(CH_3)_2$; and n is from 2 to 6.

3. A compound according to claim 1 where $R_1$ represents H, Y represents $N(CH_3)_2$ and n is 2.

4. A compound according to claim 1 where $R_1$ represents 3-Cl, Y represents $N(CH_3)_2$ and n is 2.

5. A compound according to claim 1 where $R_1$ represents 6-Cl, Y represents $N(CH_3)_2$ and n is 2.

6. A compound according to claim 1 where $R_1$ represents 6-$CH_3$, Y represents $N(CH_3)_2$ and n is 2.

7. A compound according to claim 1 or claim 2 where $R_1$ represents 9-$OCH_3$, Y represents $N(CH_3)_2$ and n is 2.

8. A compound according to claim 1 or claim 2 where $R_1$ represents 9-$CH_3$, Y represents $N(CH_3)_2$ and n is 2.

9. A compound according to claim 1 or claim 2 where $R_1$ represents 9-$OCH_2CH_3$, Y represents $N(CH_3)_2$ and n is 2.

10. A compound according to claim 1 or claim 2 where two of $R_1$ at positions 8 and 9 represent -CH=CH-CH=CH- as part of an extra benzene ring, Y represents $N(CH_3)_2$ and n is 2.

11. A process for the preparation of a compound represented by the general formula (I), as defined in claim 1, or an acid addition salt, 5- or 10-mono-N-oxide or 5,10-di-N-oxide thereof, which process comprises coupling a substituted phenazine of the general formula (II),

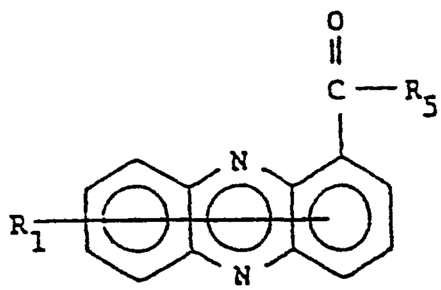

(II)

where $R_1$ is as defined in claim 1 but additionally can represent $COR_5$ at the positions 2-4 and 6-9, and $R_5$ represents Cl, Br, $OC_6H_4$-p-$NO_2$, O-(1-N-benzotriazole), 1-N-imidazole, or O-(2-N-methylpyridinium) salts, or the 5- or 10-mono-N-oxide or 5,10-di-N-oxide thereof, with a primary alkyl amine of the general formula (III),

$$NH_2(CH_2)_nY \qquad (III)$$

where n and Y are as defined in claim 1, and, if desired, converting a compound of formula (I) into an acid addition salt thereof.

12. A process according to claim 11 wherein $R_5$ in formula (II) represents Cl.

13. A process according to claim 11 wherein $R_5$ in formula (II) represents 1-N-imidazole.

14. A process according to any one of claims 11-13 wherein the coupling reaction of compound (II) with compound (III) is performed in an anhydrous solvent selected from chloroform, dimethylsulphoxide, N-methylpyrrolidone, dichloromethane and dimethylformamide, buffered with a tertiary amine.

15. A compound represented by the general formula (I) as defined in claim 1, or an acid addition salt, 5- or 10-mono-N-oxide or 5,10-di-N-oxide thereof, whenever prepared by the process according to any one of claims 11-14.

16. A compound represented by the general formula (II),

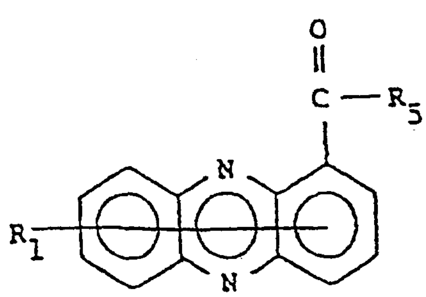

(II)

where $R_1$ is as defined in claim 1 but additionally can represent $COR_5$ at positions 2-4 and 6-9, and $R_5$ represents Cl, Br, $OC_6H_4$-p-$NO_2$, O-(1-N-benzotriazole), 1-N-imidazole, or O-(2-N-methylpyridinium) salts, or a 5- or 10- mono-N-oxide or 5,10-di-N-oxide thereof.

17. A compound of general formula (X)

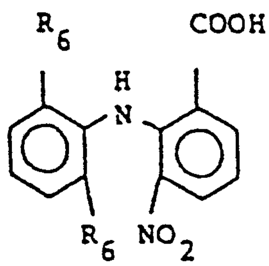

(X)

where each $R_6$ represents one of the groups halogen, $OCH_2Ph$, OPh, $CF_3$, SH, COOH, $NO_2$, lower alkyl or lower alkoxyl optionally substituted with either suitably-protected hydroxy or suitably-protected amino functions, $NHSO_2R_2$, $NHCOR_2$, or $NHCOOR_2$ or $SR_2$ (where $R_2$ is lower alkyl optionally substituted with hydroxy, amino or ether functions), but particularly where $R_6$ represents F or $OCH_3$.

18. A process for the preparation of a compound represented by the general formula (VII)

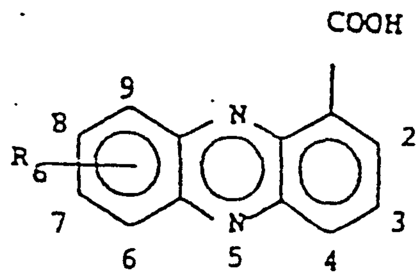

(VII)

where $R_6$ represents H or up to three of the groups halogen, SH, $OCH_2Ph$, OPh, $CF_3$, COOH, $NO_2$, lower alkyl or lower alkoxyl optionally substituted with either suitably-protected hydroxy or suitably- protected amino functions, $NHSO_2R_2$, $NHCOR_2$, $NHCOOR_2$ or $SR_2$ (where $R_2$ is lower alkyl optionally substituted with hydroxy, amino or ether functions) or two of $R_6$ at adjacent positions represent -CH=CH-CH=CH- as part of an extra benzene ring or $-O-CH_2-O-$ (methylenedioxy); which process comprises reacting a compound of formula (XII)

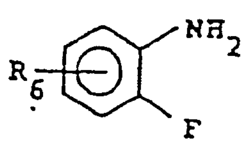

(XII)

where $R_6$ is as defined above, with a compound of the formula (V)

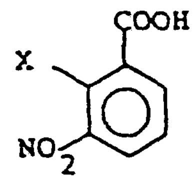

(V)

where X is Cl, Br or I, and subjecting the obtained compound of formula (XIII)

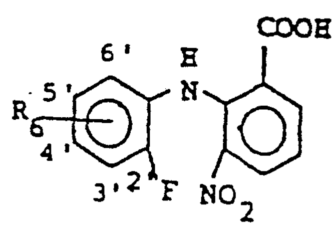

(XIII)

where $R_6$ is as defined above, to reductive cyclization.

19. A compound of the general formula (XIII)

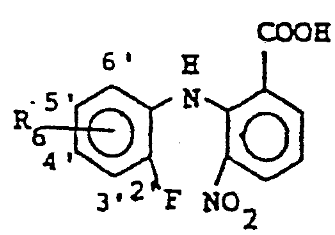

(XIII)

where $R_6$ is as defined in claim 18.

20. A pharmaceutical composition having antitumour activity which comprises at least one compound of the general formula (I) defined in claim 1, or a pharmaceutically acceptable acid addition salt, 5- or 10-mono-N-oxide or 5,10-di-N-oxide thereof, and one or more pharmaceutically acceptable carriers or diluents.

21. A pharmaceutical composition having antitumour activity which comprises a compound according to any one of claims 2 to 10 or a pharmaceutically acceptable acid addition salt, 5- or 10-mono-N-oxide or 5,10-di-N-oxide thereof, and one or more pharmaceutically acceptable carriers or diluents.

22. A compound of the general formula (I) as defined in claim 1, or a pharmaceutically acceptable acid addition salt, 5- or 10-mono-N-oxide or 5,10-di-N-oxide thereof, for use in the treatment of tumours.

23. A compound according to any one of claims 2 to 10, or a pharmaceutically acceptable acid addition salt, 5- or 10-mono-N-oxide or 5,10-di-N-oxide thereof, for use in the treatment of tumours.

CLAIMS FOR AUSTRIA

1. A process for the preparation of a compound represented by the general formula (I),

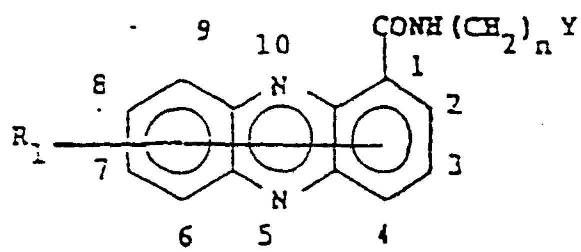

(I)

where $R_1$ represents H or up to three substituents, at positions selected from 2 to 4 and 6 to 9, wherein any two or all of the substituents may be the same or different and the substituents are selected from

lower alkyl radicals;

lower alkyl radicals substituted by one or more of the same or different substituents selected from hydroxy, amino and ether functions;

OH,

SH;

$OCH_2Ph$;

OPh;

$NO_2$;

halogen;

$CF_3$;

amino;

$NHSO_2R_2$, $NHCOR_2$, $NHCOOR_2$, $OR_2$ and $SR_2$ (where $R_2$

represents a lower alkyl radical which is unsubstituted or substituted by one or more of the same or different substituents selected from hydroxy, amino and ether functions); and

$CONH(CH_2)_{n'}Y$ (where n' and Y' are as defined below), there being a maximum of one $CONH(CH_2)_{n'}Y'$ group;

or any two of $R_1$ at adjacent positions represent -CH=CH-CH=CH- as part of an extra benzene ring or $-O-CH_2-O-$ (methylenedioxy) and the third of $R_1$ has any one of the meanings given above with the exception of an OH at position 2;

Y and Y', which may be the same or different, each represents $C(NH)NH_2$, $NHC(NH)NH_2$ or $NR_3R_4$, where each of $R_3$ and $R_4$, which may be the same or different, represents H or a lower alkyl radical unsubstituted or substituted by one or more of the same or different substituents selected from hydroxy, amino and ether functions, or $R_3$ and $R_4$, together with the nitrogen atom to which they are attached, form a heterocyclic ring; and

n and n', which may be the same or different, each represents an integer from 2 to 6;

or an acid addition salt, 5- or 10- mono-N-oxide or 5,10-di-N-oxide thereof, which process comprises coupling a substituted phenazine of the general formula (II),

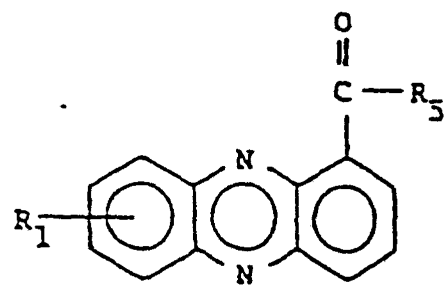

(II)

where $R_1$ is as defined above but additionally can represent $COR_5$ at the positions 2-4 and 6-9, and $R_5$ represents Cl, Br, $OC_6H_4$-p-$NO_2$, O-(1-N-benzotriazole), 1-N-imidazole, or O-(2-N-methylpyridinium) salts, or the 5- or 10-mono-N-oxide or 5,10-di-N-oxide thereof, with a primary alkyl amine of the general formula (III),

$$NH_2(CH_2)_nY \qquad (III)$$

where n and Y are as defined above, and, if desired, converting a compound of formula (I) into an acid addition salt thereof.

2. A process according to claim 1 wherein $R_5$ in formula (II) represents Cl.

3. A process according to claim 1 wherein $R_5$ in formula (II) represents 1-N-imidazole.

4. A process according to any one of claims 1 to 3 wherein the coupling reaction of compound (II) with compound (III) is performed in an anhydrous solvent selected from chloroform, dimethylsulphoxide, N-methylpyrrolidone, dichloromethane and dimethylformamide, buffered with a tertiary amine.

5. A process according to any one of claims 1 to 4 where $R_1$ represents one of lower alkyl optionally substituted with hydroxy, amino or ether functions, OH, SH, $OCH_2Ph$, OPh, $NO_2$, halogen, $CF_3$, $NH_2$, or $NHSO_2R_2$, $NHCOR_2$, $NHCOOR_2$, $OR_2$ or $SR_2$ (where $R_2$ is lower alkyl optionally substituted with hydroxy, amino or ether functions) at position 9, or where two of $R_1$ at positions 8 and 9 represent -CH=CH-CH=CH- as part of an extra benzene ring; Y represents $N(CH_3)_2$; and n is from 2 to 6.

6. A process according to any one of claims 1 to 4 where $R_1$ represents H, Y represents $N(CH_3)_2$ and n is 2.

7. A process according to any one of claims 1 to 4 where $R_1$ represents 3-Cl, Y represents $N(CH_3)_2$ and n is 2.

8. A process according to any one of claims 1 to 4 where $R_1$ represents 6-Cl, Y represents $N(CH_3)_2$ and n is 2.

9. A process according to any one of claims 1 to 4 where $R_1$ represents 6-$CH_3$, Y represents $N(CH_3)_2$ and n is 2.

10. A process according to any one of claims 1 to 5 where $R_1$ represents 9-$OCH_3$, Y represents $N(CH_3)_2$ and n is 2.

11. A process according to any one of claims 1 to 5 where $R_1$ represents 9-$CH_3$, Y represents $N(CH_3)_2$ and n is 2.

12. A process according to any one of claims 1 to 5 where $R_1$ represents 9-$OCH_2CH_3$, Y represents $N(CH_3)_2$ and n is 2.

13. A process according to any one of claims 1 to 5 where two of $R_1$ at positions 8 and 9 represent -CH=CH-CH=CH- as part of an extra benzene ring, Y represents $N(CH_3)_2$ and n is 2.

14. A process for the preparation of a compound represented by the general formula (II),

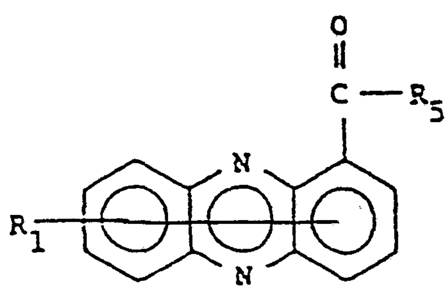

(II)

where $R_1$ is as defined in claim 1 but additionally can represent $COR_5$ at positions 2-4 and 6-9, and $R_5$ represents Cl, Br, $OC_6H_4$-p-$NO_2$, O-(1-N-benzotriazole), 1-N-imidazole, or O-(2-N-methylpyridinium) salts, or a 5- or 10-mono-N-oxide or 5,10-di-N-oxide thereof, which process comprises reacting a compound of the general formula (VIII)

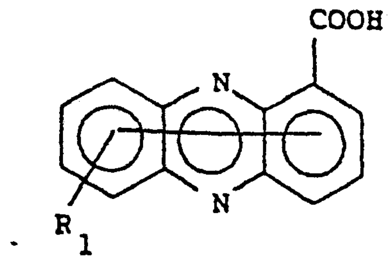

(VIII)

where $R_1$ is as defined above in claim 1 but additionally can represent COOH at positions 2 to 4 and 6 to 9, or a 5- or 10- mono-N-oxide or 5,10-di-N-oxide thereof, with a chlorinating agent, a brominating agent, tris(4-nitrophenyl)phosphite, 1,1'-carbonyldiimidazole, bis(1-N-benzotriazole)carbonate or a 2-chloro-N-methylpyridinium salt.

15. A process for the preparation of a compound represented by the general formula (X)

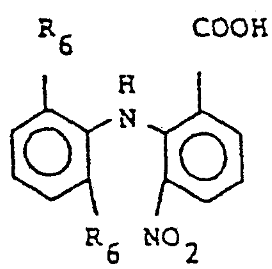

(X)

where each $R_6$ represents one of the groups halogen, SH, $OCH_2Ph$, OPh, $CF_3$, COOH, $NO_2$, lower alkyl or lower alkoxyl optionally substituted with either suitably-protected hydroxy or suitably-protected amino functions, $NHSO_2R_2$, $NHCOR_2$, $NHCOOR_2$ or $SR_2$ (where $R_2$ is lower

alkyl optionally substituted with hydroxy, amino or ether functions), but particularly where $R_6$ represents F or $OCH_3$, which process comprises reacting a compound of formula (IX)

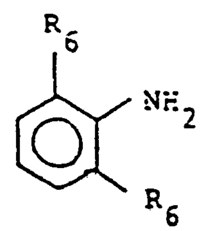

(IX)

wherein $R_6$ is as defined above, with a compound of formula (V)

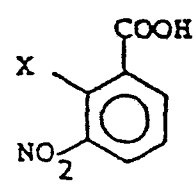

(V)

wherein X is Cl, Br or I.

16. A process for the preparation of a compound represented by the general formula (VII)

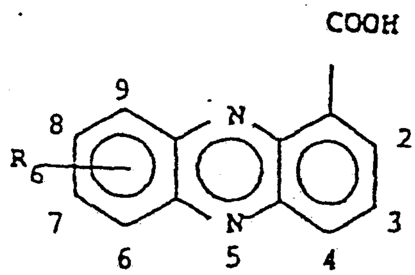

(VII)

where $R_6$ represents H or up to three of the groups halogen, SH, $OCH_2Ph$, OPh, $CF_3$, COOH, $NO_2$, lower alkyl or lower alkoxyl optionally substituted with either suitably-protected hydroxy or suitably- protected amino functions, $NHSO_2R_2$, $NHCOR_2$, $NHCOOR_2$ or $SR_2$ (where $R_2$ is lower alkyl optionally substituted with hydroxy, amino or ether functions) or two of $R_6$ at adjacent positions represent -CH=CH-CH=CH- as part of an extra benzene ring or $-O-CH_2-O-$ (methylenedioxy); which process comprises reacting a compound of formula (XII)

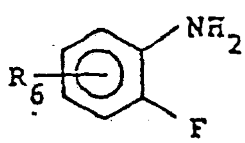

(XII)

where $R_6$ is as defined above, with a compound of the formula (V)

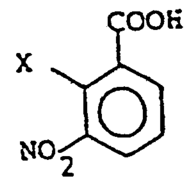

(V)

where X is Cl, Br or I, and subjecting the obtained compound of formula (XIII)

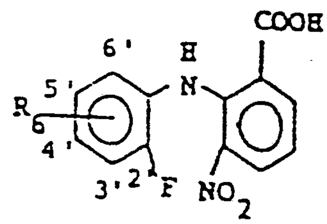

(XIII)

where $R_6$ is as defined above, to reductive cyclization.

17. A process for the preparation of a compound represented by the general formula (XIII)

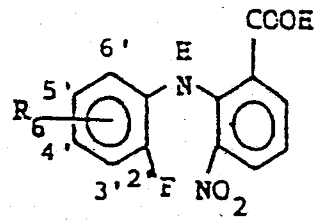

(XIII)

where $R_6$ is as defined in claim 16, which process comprises reacting a compound of formula (XII)

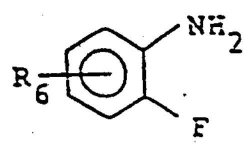

(XII)

where $R_6$ is as defined above, with a compound of the formula (V)

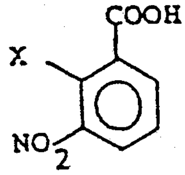

(V)

where X is Cl, Br or I.

18. A process for the preparation of a pharmaceutical composition having antitumour activity which comprises bringing into admixture or conjunction at least one compound of the general formula (I) defined in claim 1, or a pharmaceutically acceptable acid addition salt, 5- or 10-mono-N-oxide or 5,10-di-N-oxide thereof, and one or more pharmaceutically acceptable carriers or diluents.